# EUROPÄISCHE PATENTANMELDUNG

(11) **EP 0 669 314 A1**
(43) Veröffentlichungstag der Anmeldung: **30.08.1995**
(21) Anmeldenummer: 95101989.2
(22) Anmeldetag: 14.02.1995
(51) Int. Cl.: C07C 209/26, C07C 211/35

(54) **Verfahren zur Herstellung substituierter cis-Cyclohexylamine**

(30) Priorität: 23.02.1994 DE 4405728
(71) Anmelder: Hoechst Schering AgrEvo GmbH, 13509 Berlin (DE)
(72) Erfinder: Ressel, Hans-J., Dl., D-65795 Hattersheim (DE)

(57) **Zusammenfassung**

Die Erfindung betrifft ein Verfahren zur Herstellung von cis-konfigurierten Verbindungen der Formel, 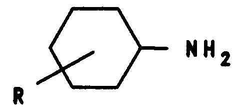
in welcher R für einen gegebenenfalls substituierten aliphatisch acyclischen, alicyclischen, aromatischen oder heterocyclischen Rest steht, bei welchem man ein entsprechendes Cyclohexanon in Gegenwart eines Edelmetallborid-Katalysators oder -Katalysatorgemisches mit Ammoniak und Wasserstoff umsetzt.

## Beschreibung

Substituierte cis-Cyclohexylamine sind wertvolle Zwischenprodukte. Sie finden beispielsweise bei der Herstellung der aus PCT/EP93/00536 bekannten insektizid, akarizid und fungizid wirksamen substituierten 4-Amino- und 4-Alkoxypyrimidine Verwendung.

Es wurde ein neues Verfahren zur Herstellung von cis-Cyclohexylaminen durch reduktive Aminierung entsprechender Cyclohexanone gefunden, das sich durch hohe Stereoselektivität auszeichnet.

Die Erfindung betrifft ein Verfahren zur Herstellung von cis-konfigurierten Verbindungen der Formel I, 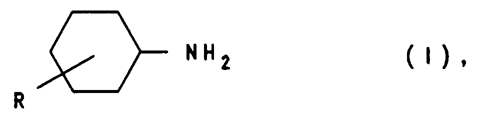
in welcher R für einen gegebenenfalls substituierten aliphatisch acyclischen, alicyclischen, aromatischen oder heterocyclischen Rest steht, bei welchem man ein substituiertes Cyclohexanon der Formel II 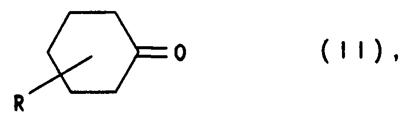
in welcher R wie oben definiert ist, in Gegenwart eines Edelmetallborid-Katalysators oder -Katalysatorgemisches mit Ammoniak und Wasserstoff umsetzt.

Es ist bekannt, daß Cyclohexanone reduktiv aminiert werden können. Dabei führen Raney-Nickel oder -Kobalt sowie Ruthenium-(O)-Katalysatoren zu geringer cis-Selektivität (60 bis 65 % mit Ni/Co bzw. 75 bis 78 % mit Ru; vgl.

Beispiel 6).

Bei Katalyse durch Platin- oder Palladium-(O)-Katalysatoren beobachtet man neben niedriger cis-Selektivität (40 bzw. 50-60 %) auch Nebenreaktionen, wie die Bildung von Dicyclohexylaminen der Formel III 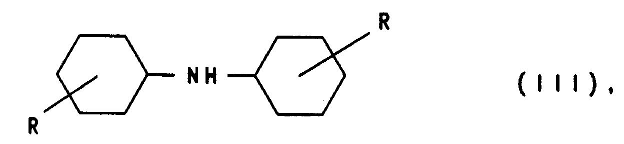
und von Alkoholen der Formel (IV) (durch Reduktion der Keto-Funktion), 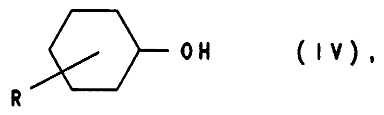
mit der Folge, daß die chemische Ausbeute an primärem Amin der Formel I abnimmt (auf 50 bis 60 %, vgl. Beispiel 3).

Die Verwendung von Rhodium-(O)-Katalysatoren (vgl. Beispiel 4) führt zu besseren chemischen Ausbeuten bei guter cis-Selektivität (90 % bzw. 85 bis 90 %). Nachteilig ist jedoch der erhebliche Abfall der cis-Selektivität bei der Wiederverwendung dieser Katalysatoren und ihr hoher Preis bei gleichzeitig niedriger Rückgewinnungsrate für das Edelmetall. [Yada, S., et al., Coll-Humanit. Sci., Nihon Univ., Tokyo 156, Nippon Kagaku Kaishi (4), 641-7 (1989)].

Andere Herstellungsmethoden für substituierte cis-Cyclohexylamine sind beschrieben, doch werden die Zielverbindungen entweder in niedrige Ausbeuten erhalten, oder es werden teure, nur für Laborverfahren geeignete Reagenzien benötigt; z.B. Reduktion des Oxims mit Natrium in Ethanol [Ramaligan, K., et al, Indian J. of Chem., Vol. 10 (1972), 366-369] oder Reduktion eines N-Benzylimins in mehreren Stufen mit Pd-(O)-Katalysatoren oder mit sterisch gehinderten Borhydriden, wie LiBH (S-butyl)₃ [Hutchins, R.O., Su, W.-Y., Tetrah. Lett., Vol. 25, No. 7 (1984), 695-698].

Die reduktive Aminierung von Keto-Funktionen anderer Moleküle unter Metallborid-Katalyse ist z.B. für Kobaltborid [CS 239 418] bzw. Nickelborid [SU 1204 617-A] beschrieben, wobei jedoch keine stereochemische Selektivität zu beachten war. Im Falle substituierter Cyclohexanone konnte bei Verwendung dieser nickelhaltigen Katalysatoren keine hohe cis-Selektivität erzielt werden. Die Ergebnisse liegen im Bereich der Raney-Metall-Katalyse (60 bis 70 % cis; vgl. Beispiel 5).

Überraschenderweise führen die erfindungsgemäßen Edelmetallborid-Katalysatoren, insbesondere die Palladiumborid-Katalysatoren im Vergleich zu herkömmlichen Katalysatoren zu deutlicheren Vorteilen:

Bei der reduktiven Aminierung substituierter Cyclohexanone werden hohe chemische Ausbeuten (> 90 %) und große cis-Selektivität (bis zu 90 %) erzielt, wobei die Reaktion im Gegensatz zur Verwendung gebräuchlicher Katalysatoren ohne großen apparativen Aufwand schon bei ca. 20 bar durchgeführt werden kann. Desweiteren ist eine sehr gute Rückgewinnung des Edelmetalls gewährleistet. Der Katalysator ist stabil und kann ohne Aktivitätsverlust vielfach (in der Regel bis zu 10 fach) wieder eingesetzt werden.

Bevorzugte Bedeutungen von R sind (C₁-C₁₂)-Alkyl, (C₃-C₈)-Cycloalkyl, (C₃-C₈)-Cycloalkyl-(C₁-C₄)-alkyl, (C₁-C₈)-Alkoxy,(C₃-C₈)-Cycloalkoxy, (C₁-C₄)-Alkoxy-(C₁-C₄)-alkyl, (C₃-C₈)-Cycloalkyl-(C₁-C₄)-alkoxy, Tri-(C₁-C₈)-alkylsilyl, vorzugsweise Dimethyl-(C₁-C₈)alkylsilyl oder Triethylsilyl, Di-(C₁-C₈)-alkyl-(C₃-C₈)-cycloalkylsilyl, vorzugsweise Dimethylcyclohexylsilyl, Di-(C₁-C₈)-alkyl-(phenyl-(C₁-C₄)-alkyl)-silyl, vorzugsweise Dimethyl-(phenyl-(C₁-C₄)-alkyl)-silyl, Di-(C₁-C₈)-alkyl-(C₁-C₄)-halogenalkylsilyl, vorzugsweise Dimethyl-(C₁-C₄)-halogenalkylsilyl, Dimethylphenylsilyl, (C₁-C₄)-Halogenalkyl, Halogen, (C₁-C₄)-Halogenalkoxy, Heteroaryl, Aryl, Aryl-(C₁-C₄)-alkyl, Benzyloxy, Benzyloxy-(C₁-C₄)-alkyl, Benzylthio, Arylthio oder Aryloxy, wobei Aryl bzw. Phenyl in den sieben letztgenannten Resten unsubstituiert oder mit einem oder mehreren, vorzugsweise zwei Substituenten versehen sein können und diese Substituenten gleich oder verschieden sind und jeweils (C₁-C₈)-Alkyl, (C₃-C₈)-Cycloalkyl (C₁-C₄)-Halogenalkyl, vorzugsweise Trifluormethyl, Halogen, (C₁-C₄)-Dialkylamino, (C₁-C₄)-Alkylthio, (C₁-C₈)-Alkoxy, (C₁-C₄)-Halogenalkoxy, (C₁-C₄)-Alkoxy-(C₁-C₄)-alkoxy, H₅C₂-O-[CH₂-CH₂-O-]ₓ mit x = 2, 3 oder 4, vorzugsweise 2, 2-(Tetrahydro-2H-pyran-2-yloxy)-ethoxy, (C₂-C₈)-Alkenyl, (C₂-C₈)-Alkinyl, Benzyloxy, welches im Phenylrest gegebenenfalls einen oder zwei gleiche oder verschiedene Substituenten aus der Reihe (C₁-C₄)-Alkyl, (C₁-C₄)-Halogenalkyl, (C₁-C₄)-Alkoxy, (C₁-C₄)-Halogenalkoxy und Halogen trägt, Tri-(C₁-C₄)-alkylsilylmethoxy, vorzugsweise Dimethyl-(C₁-C₄)-alkylsilylmethoxy, (C₃-C₈)-Cycloalkyl-(C₁-C₄)-alkoxy, 1,3-Dioxolan-2-ylmethoxy, Tetrahydrofuran-2-ylmethoxy oder Tetrahydro-2H-pyran-2-ylmethoxy bedeuten können.

R bedeutet insbesondere (C₃-C₈)-Alkyl, ganz besonders bevorzugt tert.Butyl, Cyclopentyl, Cyclohexyl, Phenyl oder Phenoxy, wobei die beiden letztgenannten Reste unsubstituiert oder mit einem oder zwei Substituenten versehen sein können, die gleich oder verschieden sein können und diese Fluor, Chlor, Brom, (C₁-C₄)-Alkyl, (C₁-C₄)-Alkoxy, Trifluormethyl, (C₁-C₂)-Halogenalkoxy, Cyclohexyl, 2-Ethoxyethoxy, Methylthio oder Dimethylamino bedeuten.

Unter "Halogen" ist ein Fluor-, Chlor-, Brom- oder lodatom vorzugsweise ein Fluor-, Chlor- oder Bromatom zu verstehen, unter dem Ausdruck "(C₁-C₄)-Alkyl" ein unverzweigter oder verzweigter Kohlenwasserstoffrest mit 1-4 Kohlenstoffatomen, wie z.B. der Methyl-, Ethyl-, Propyl-, 1-Methylethyl-, 1-Methylpropyl-, 2-Methylpropyl- oder 1 -Dimethylethylrest,
unter dem Ausdruck "(C₁-C₈)-Alkyl" die vorgenannten Alkylreste sowie z.B. der Pentyl, 2-Methylbutyl- oder der 1,1-Dimethylpropylrest, der Hexyl-, Heptyl-, Octylrest oder 1,1,3,3-Tetramethylbutyl;
unter dem Ausdruck "(C₁-C₁₂)-Alkyl" die vorgenannten Alkylreste sowie z.B. der Nonyl-, Decyl-, Undecyl- oder Dodecylrest;
unter dem Ausdruck "(C₃-C₈)-Cycloalkyl" die Cyclopropyl-, Cyclobutyl-, Cyclopentyl-, Cyclohexyl-, Cycloheptyl- oder Cyclooctylgruppe;
unter dem Ausdruck (C₁-C₄)-Alkoxy" eine Alkoxygruppe, deren Kohlenwasserstoffrest die unter dem Ausdruck "(C₁-C₄)-Alkyl" angegebene Bedeutung hat;
unter dem Ausdruck "(C₃-C₈)-Cycloalkoxy" eine Cycloalkoxygruppe, deren Kohlenwasserstoffrest die unter (C₃-C₈)-Cycloalkyl" angegebene Bedeutung hat;
unter dem Ausdruck (C₁-C₄)-Alkylthio" eine Alkylthiogruppe, deren Kohlenwasserstoffrest die unter dem Ausdruck (C₁-C₄)-Alkyl" angegebene Bedeutung hat;
unter dem Ausdruck "(C₁-C₄)-Halogenalkoxy" eine Halogenalkoxygruppe, deren Halogen-Kohlenwasserstoffrest die unter dem Ausdruck "(C₁-C₄)-Halogenalkyl" angegebene Bedeutung hat,
unter dem Ausdruck "(C₁-C₄)-Alkoxy-(C₁-C₄)alkyl" beispielsweise eine 1-Methoxyethylgruppe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe;
unter dem Ausdruck "(C₁-C₄)-Alkylthio-(C₁-C₄)alkyl" beispielsweise Methylthiomethyl, Ethylthiomethyl, Propylthiomethyl, 2-Methylthioethyl, 2-Ethylthioethyl oder 3-Methylthiopropyl;
unter dem Ausdruck "(C₁-C₄)-Alkylamino" eine Alkylaminogruppe, deren Kohlenwasserstoffrest die unter dem Ausdruck (C₁-C₄)-Alkyl" angegebene Bedeutung hat, bevorzugt die Ethyl- und Methylaminogruppe;
unter dem Ausdruck "Di-(C₁-C₄)-alkylamino" eine Dialkylaminogruppe, deren Kohlenwasserstoffreste die unter dem Ausdruck (C₁-C₄)-Alkyl" angegebene Bedeutung haben, bevorzugt die Dimethyl- und Diethylaminogruppe;
unter dem Ausdruck "(C₁-C₄)-Halogenalkyl" eine unter dem Ausdruck "(C₁-C₄)-Alkyl" genannte Alkylgruppe, in der eines oder mehrere Wasserstoffatome durch die obengenannten Halogenatome, bevorzugt Chlor oder Fluor, ersetzt wird, wie beispielsweise die Trifluormethylgruppe, die 2,2,2-Trifluorethylgruppe, die Chlormethyl-, Fluormethylgruppe, die Difluormethylgruppe oder die 1,1,2,2-Tetrafluorethylgruppe;
unter dem Ausdruck "C₃-C₈)-Cycloalkyl-(C₂-C₄)-alkyl" eine der oben genannten (C₁-C₄)-Alkylgruppen, die mit einer der oben genannten (C₃-C₈)-Cycloalkylgruppen substituiert ist, beispielsweise Cyclohexylmethyl, Cyclohexylethyl, Cyclohexylpropyl, Cyclohexylbutyl oder 1-Cyclohexyl-1-methyl-ethyl;
unter dem Ausdruck "Phenyl-(C₁-C₄)-alkyl" eine der oben genannten (C₁-C₄)-Alkylgruppen, die mit einer Phenylgruppe substitutiert ist, beispielsweise die Benzyl-, die 2-Phenylethyl-, die 1-Phenylethyl-, die 1-Methyl-1-phenylethylgruppe, die 3-Phenylpropyl-, die 4-Phenylbutylgruppe oder die 2-Methyl-2-phenyl-ethylgruppe;
unter dem Ausdruck "Aryl" beispielsweise, Phenyl, Naphthyl oder Biphenylyl, vorzugsweise Phenyl;
unter dem Ausdruck "Heteroaryl" einen Arylrest, worin mindestens eine CH-Gruppe durch N ersetzt ist und/oder mindestens zwei benachbarte CH-Gruppen durch S, NH oder O ersetzt sind;
unter dem Ausdruck "Benzyloxy-(C₁-C₄)-alkyl" eine (C₁-C₄)-Alkylgruppe mit den oben angegebenen Bedeutungen die mit einer Benzyloxygruppe substituiert ist, z.B. die Benzyloxymethyl- oder die 2-(Benzyloxy)-ethyl-Gruppe;
unter dem Ausdruck "(C₃-C₈)-Cycloalkyl-(C₁-C₄)-alkoxy" eine (C₁-C₄)-Alkoxygruppe mit den oben angegebenen Bedeutungen, die mit einer (C₃-C₈)-Cycloalkylgruppe mit den oben angegebenen Bedeutungen substituiert ist, z.B. die Cyclopropylmethyl- oder die Cyclohexylmethylgruppe;
unter dem Ausdruck "Tri-(C₁-C₈)-alkylsilyl" eine Trialkylsilylgruppe die vorzugsweise zwei Methylgruppen und eine (C₁-C₈)-Alkylgruppe mit den oben angegebenen Bedeutungen trägt, z.B. die Trimethylsilyl-, die Dimethylethylsilyl oder die Dimethyloctylsilylgruppe;
unter dem Ausdruck "Di-(C₁-C₈)-alkyl-(C₁-C₄)-halogenalkylsilyl" einen Silylrest, der vorzugsweise zwei Methylgruppen und einen (C₁-C₄)-Halogenalkylrest mit den unter dem Ausdruck (C₁-C₄)-Halogenalkyl angegebenen Bedeutungen trägt, z.B. der Dimethyl-3,3,3-trifluorpropylsilyl-Rest;
unter dem Ausdruck (C₁-C₄)-Alkoxy-(C₁-C₄)-alkoxy" z.B. die Ethoxymethoxy-, 2-Ethoxy-ethoxy-, 2-Butoxyethoxy- oder 2-Methoxyethoxy-Gruppe; unter dem Ausdruck "(C₂-C₈)-Alkenyl" z.B. die Allyl-, 1-Methylallyl-, 2-Butenyl-oder 3-Methyl-2-butenyl-Gruppe;
unter dem Ausdruck (C₂-C₈)-Alkinyl" z.B. die Propargyl-, 2-Butinyl- oder 2-Pentinyl-gruppe;
unter dem Ausdruck "Tri-(C₁-C₄)-alkylsilylmethoxy" ein Trialkylsilylmethoxyrest mit vorzugsweise 2 Methylgruppen, worin die (C₁-C₄)-Alkylgruppe die oben angegebenen Bedeutungen hat;
unter dem Ausdruck Di-(C₁-C₈)-alkyl-phenyl-(C₁-C₄)-alkylsilyl ein Trialkylsilylrest mit vorzugsweise zwei Methylgruppen, bei dem eine Alkylgruppe die oben für den Ausdruck "Phenyl-(C₁-C₄)-alkyl" angegebenen Bedeutungen hat, vorzugsweise die Dimethylbenzylsilyl-Gruppe.

Die oben gegebene Erläuterung gilt entsprechend für Homologe bzw. deren abgeleitete Reste.

R steht vorzugsweise in 4-Position.

Die Reaktionstemperatur liegt zwischen 0 und + 100 °C, vorzugsweise bei +20 bis +60 °C; der Wasserstoffdruck zwischen 1 und 100 bar, vorzugsweise zwischen 5 und 50 bar.

Ammoniak wird vorzugsweise in 1,0- bis 6-facher molarer Menge verwendet.

Als Lösungsmittel werden bevorzugt niedere aliphatische oder cyclische Alkohole, Äther, Ester, Kohlenwasserstoffe, Wasser, Ammoniak, Eisessig oder Gemische daraus verwendet; es kann auch ohne Lösungsmittel gearbeitet werden.

Geeignete Edelmetallboride sind beispielsweise die Boride von Ru, Rh, Pt und Pd oder deren Mischungen, bevorzugt ist Palladiumborid. Der Edelmetallborid-Katalysator wird vorzugsweise auf einem geeigneten Träger, wie Aktivkohle, Aluminiumoxid, Kieselgur, Zeolith, Calciumcarbonat, eingesetzt.

Als Ausgangsmaterialien für die Zubereitung des Katalysators kommen herkömmliche Edelmetall-Salze in Frage, deren Löslichkeit in Wasser und/oder

niederen aliphatischen Alkoholen oder anderen organischen Lösungsmittel gegeben ist, wie Chloride, Bromide, Acetate.

Der Katalysator kann durch Borhydrid-Reduktion der Edelmetallsalze, z.B. analog einer Vorschrift zur Darstellung von Nickelborid hergestellt werden: [C.A. Brown, H.C. Brown, J.A.C.S. 85 (1963), 1003 und 1005]. Als Lösungsmittel kommen hierbei Wasser, niedrige aliphatische Alkohole und andere organische Lösungsmittel, wie z.B. Tetrahydrofuran, 1,4-Dioxan, Acetonitril und deren Mischungen zur Verwendung.

Unter den Bedingungen des erfindungsgemäßen Verfahrens können einige der genannten Reste R abhydriert werden (z.B. R = Benzyloxy unter Bildung von R = Hydroxy). Die so gebildeten Reste R können in an sich bekannter Weise in die Reste R der Ausgangsverbindungen zurückverwandelt werden.

Die folgenden Beispiele dienen zur Erläuterung der Erfindung, ohne daß diese darauf beschränkt wäre.

Beispiel für die Herstellung des Katalysators

0,5 g Palladiumacetat werden in 50 ml Ethanol gelöst und mit 5 g Aktivkohle (Riedel de Haen, No. 18003) versetzt. Bei Raumtemperatur wird langsam eine Lösung von 2,0 g Natriumborhydrid in 50 ml Wasser zugetropft. Die Reduktion erfolgt spontan unter Entwicklung von Wasserstoff. Man rührt noch für 30 min. nach und filtriert den Katalysator ab. Es wird 3 mal mit Ethanol und 2 mal mit Wasser gewaschen. Der wasserfeuchte Katalysator ist einsatzfertig.

Allgemein führt man die reduktive Aminierung so durch, daß man das Keton im Lösungsmittel löst, den Katalysator zugibt und anschließend in einen Stahlautoklaven überführt. Die erforderliche Ammoniak-Menge wird dann über ein Ventil zugeführt und das Reaktionsgemisch unter Wasserstoff-Atmosphäre auf die erwünschte Temperatur eingestellt. Nun wird bis zu Beendigung der Wasserstoffaufnahme gerührt.

Das Produkt wird üblicherweise aus dem Rohgemisch isoliert, indem man nach Belüften mit Stickstoff den Katalysator abfiltriert und die Niedrigsieder

(Lösungsmittel, NH₃) abtrennt. Eine Reinigung kann durch Feindestillation erfolgen.

Bevorzugt wird die Reaktion so geführt, daß man das Keton (evtl. gelöst in Lösungsmittel) langsam bei den erforderlichen Reaktionsbedingungen zum Katalysator/Ammoniak/Wasserstoff/Lösungsmittel-Gemisch zudosiert. Andere Reaktorformen wie Schlaufenreaktoren u.a. zur kontinuierlichen Verfahrensweise sind ebenfalls vorteilhaft anwendbar.

### Beispiel 1

In einem Stahlautoklaven mit Begasungsrührer werden bei 50 °C 2 Liter Isopropanol, 204 g Ammoniak sowie der Palladiumborid-Kohle-Katalysator, hergestellt nach dem oben beschriebenen Verfahren aus 1,5 g Palladiumacetat in 150 ml Ethanol/50 ml Wasser, 15 g A-Kohle, 6 g NaBH₄ in 150 ml Wasser, vorgelegt.

Nach Aufdrücken einer Wasserstoffatmosphäre von 20 bar wird nun über die Dauer von 3 Stunden eine Lösung von 308,4 g 4-tert.-Butylcyclohexanon in einem Liter Isopropanol zudosiert, wobei der Druck durch weitere Zufuhr von Wasserstoff konstant gehalten wird. Im Anschluß läßt man noch für eine Stunde nachreagieren, entspannt und belüftet mit Stickstoff.

Unter Stickstoff-Atmosphäre wird der Katalysator über eine Drucknutsche abfiltriert und 2 mal mit Isopropanol gewaschen; er kann direkt wiederverwendet werden. Das Filtrat wird am Rotationsverdampfer eingeengt. Das so erhaltene Rohprodukt, 309,2 g, wird über GC-Analyse charakterisiert.

Es besteht zu 94,4 % aus 4-tert.-Butylcyclohexylamin, 1,2 %
4-tert.Butylcyclohexanol und 4,4 % Di-(4-tert.Butylcyclohexyl)-amin.
4-tert.Butylcyclohexylamin liegt zu 89,8 % in der cis-Form und zu 10,2 % in der trans-Form vor.

### Beispiel 2

100 g 4-tert.Butylcyclohexanon werden in 100 ml Methanol gelöst und zusammen mit 10 g Palladiumborid-Kohle (wie Beispiel 1) und einer Lösung von 60 g Ammoniak in 300 ml Methanol in einem Schüttelautoklaven bei 50 °C und einem Wasserstoffdruck von 50 bar hydriert, bis die Wasserstoffaufnahme beendet ist. Nach Abkühlen, Entspannen und Belüften mit Stickstoff wird der Katalysator abfiltriert und mit Methanol 3 mal gewaschen. Die Filtrate werden eingedampft. Das verbleibende Rohprodukt (100,5 g) zeigt nach GC-Analyse folgende Zusammensetzung:
92,5 % 4-tert.Butylcyclohexylamin, 0,8 % 4-tert.Butylcyclohexanol, 6,0 % Di-(4-tert.Butylcyclohexyl)amin. Das 4-tert.Butylcyclohexylamin liegt zu 89,1 % in der cis-Form und 10,9 % in der trans-Form vor.

### Beispiel 3 (Vergleichsbeispiel)

(Verfahren analog Beispiel 2). Es werden 100 g 4-tert.Butylcyclohexanon in 100 ml Methanol gelöst und zusammen mit 10 g käuflichem 5 %igem Palladium-Kohle-Katalysator (Degussa E 101 Nn/W) und einer Mischung aus 60 g Ammoniak/300 ml Methanol in einem Schüttelautoklaven bei 50 °C/50 bar Wasserstoffdruck hydriert.
Nach Aufarbeitung werden 96,3 g Rohprodukt erhalten, das nach GC-Analyse zu 50,4 % aus 4-tert.Butylhexylamin, 1,3 % 4-tert.Butylcyclohexanol und 48,3 % aus Di-(4-tert.Butylcyclohexyl)-amin besteht. 4-tert.-Butylcyclohexylamin liegt zu 60,5 % in der cis-Form und 39,5 % in der trans-Form vor.

### Beispiel 4 (Vergleichsbeispiel)

Verfahren analog Beispielen 2 und 3, jedoch unter Verwendung von 10 g 5 %igem Rhodium-Kohle-Katalysator (Degussa G 101, XN/D).
Rohprodukt: 101 g, It. GC-Analyse 90,3 % 4-tert.Butylcyclohexylamin, 3,5 % 4-tert.Butylcyclohexanol, 4,8 % Di-(4-tert.Butylcyclohexyl)-amin.
4-tert.Butylcyclohexylamin liegt zu 86,8 % in der cis-Form und 13,2 % in der trans-Form vor.

### Beispiel 5 (Vergleichsbeispiel)

Verfahren analog Beispiel 2, jedoch unter Verwendung von 5 g Kobaltborid (hergestellt nach der Vorschrift von C.A. Brown; s.o.).
Das Produkt (99,8 g) enthält 90,5 % 4-tert.Butylcyclohexylamin, das zu 71,8 % als cis und zu 28,2 % in trans-Form vorliegt.

### Beispiel 6 (Vergleichsbeispiel)

Verfahren analog Beispiel 2, jedoch unter Verwendung eines Ruthenium-(O)-Kohle-Katalysators (10 g) (Typ Degussa H 101 B/W, 5%ig);
Bedingungen: 80 bar H₂-Druck; 50 °C.
Das Produkt (95,6 g) enthält 88,2 % 4-tert.Butylcyclohexylamin, das zu 78,2 % in der cis-Form und zu 21,8 % in der trans-Form vorliegt.

## Patentansprüche

1. Verfahren zur Herstellung von cis-konfigurierten Verbindungen der Formel I 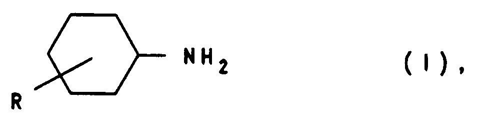 in welcher R für einen gegebenenfalls substitutierten aliphatisch acyclischen, alicyclischen, aromatischen oder heterocyclischen Rest steht, bei welchem man ein substituiertes Cyclohexanon der Formel II 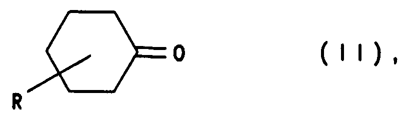 in welcher R wie oben definiert ist, in Gegenwart eines Edelmetallborid-Katalysators oder -Katalysatorgemisches mit Ammoniak und Wasserstoff umsetzt.

2. Verfahren gemäß Anspruch 1, bei welchem Palladiumborid als Katalysator verwendet wird.

3. Verfahren gemäß Anspruch 1 oder 2, dadurch gekennzeichnet, daß der Borid-Katalysator auf einem geeigneten Träger aufgebracht ist.

4. Verfahren gemäß einem der Ansprüche 1 bis 3, dadurch gekennzeichnet, daß die Reaktion in einem Lösungsmittel durchgeführt wird.

5. Verfahren gemäß einem der Ansprüche 1 bis 4, dadurch gekennzeichnet, daß das Verfahren ohne Lösungsmittel durchgeführt wird.

6. Verfahren gemäß einem der Ansprüche 1 bis 5, dadurch gekennzeichnet, daß man die Reaktion bei einem Druck von 1 bis 100 bar, vorzugsweise 5 bis 50 bar durchführt.

7. Verfahren gemäß einem der Ansprüche 1 bis 6, dadurch gekennzeichnet, daß man die Reaktion bei Temperaturen von 0 bis 100 °C, vorzugsweise zwischen +20 und +60 °C durchführt.

8. Verfahren gemäß einem der Ansprüche 1 bis 7, dadurch gekennzeichnet, daß man das Verfahren semikontinuierlich oder kontinuierlich durchführt und dabei das Keton der Formel II während des Verfahrens zudosiert.
